# EUROPEAN PATENT SPECIFICATION

(11) **EP 3 597 268 B1**
(45) Date of publication and mention of the grant of the patent: **28.10.2020**
(21) Application number: 18184512.4
(22) Date of filing: 19.07.2018
(51) Int. Cl.: A61N 5/06

(54) **IRRADIATING DEVICE AND IRRADIATION METHOD**
BESTRAHLUNGSVORRICHTUNG UND BESTRAHLUNGSVERFAHREN
DISPOSITIF ET PROCÉDÉ D'IRRADIATION

(43) Date of publication of application: 22.01.2020
(73) Proprietor: JK-Holding GmbH, 53578 Windhagen (DE)
(72) Inventor: GERSTENMEIER, Jürgen, 53578 Windhagen (DE)
(74) Representative: Sparing, Rolf Klaus

(56) References cited:
- WO-A1-2010/004500
- DE-A1- 19 502 983
- DE-A1- 19 640 118
- DE-U1- 8 701 889
- DE-U1- 29 516 572
- US-A1- 2004 088 028

## Description

### Technical Field

The present invention relates to an irradiating device. In particular, the present invention relates to a device allowing directing irradiating radiation to a subject, for example to the whole body of a human being, even more specifically to those parts of the body of a human being which are not exposed at all, or are exposed to a minor extent, only, to said radiation irradiated by conventional irradiating devices. Non-restricting examples of those parts of a human being not targeted by radiation irradiated by conventional irradiating devices are the scalp, the shoulders, the areas between the arms and the upper part of the body and the areas between the legs. Without restricting the invention, the present specification is exemplarily explaining irradiating devices directing irradiating radiation to the shoulders of a subject as, for example, of a human being. The present invention also relates to a non-therapeutical method allowing irradiating radiation to usually unirradiated parts of the body of a human being.

### Description of the Prior Art

Devices and methods for irradiating radiation to a subject, for example to the body of a human being, are known from the prior art and are practically used for a number of purposes.

Tanning of the human body, i. e. an activation of melanin generation and a conversion of melanin into the dark (brown) form of the pigment naturally occurring in the human skin, is effected by irradiating, to the skin of a subject/ human being, radiation having a wavelength λ in the range of from 280 to 315 nm (UV-B radiation) and/or radiation having a wavelength of from 315 to 400 nm (UV-A radiation). Tanning may serve purposes in the cosmetic and/or medical field(s). The number of applications is steadily increasing.

Moreover, radiation having wavelengths in the visible and near IR (infra-red) range (from 400, or better from > 550 nm to 850 nm), when irradiated onto the human skin, is capable of activating the synthesis of beneficial compounds for the human skin's nourishing, rejuvenation and regeneration, e. g. collagen, elastin, keratin, and hyaluronic acid, as is known from, e. g., EP 2 500 060 A1, from US 2012/150,265 A1 and from WO 2010/070,277 A1.

Devices capable of irradiating UV-B, UV-A, visible red and near infrared light to the whole of, or a part of the body of a subject, e. g. of a human being, in the course of a phototherapy session have beneficial effects on the user standing in a surrounding vertical light-transmissive (e. g. acrylic) user protection wall of the device or lying on a light-transmissive (e. g. acrylic) user support of the device having UV-B, UV-A, and/or visible red and infra-red light-emitting light sources mounted outside the user protection or below the user support. The latter devices may also have a hingably connected canopy that contains further UV-B, UV-A, and/or visible red and infra-red light sources mounted above a light-transmissive protective layer, thereby allowing the light sources to irradiate UV-B, UV-A and/or visible red and infra-red light to the face and back sides of the subject/user.

The document DE 102 33 984 A1 relates to a solarium which comprises two semi-cylinder shape units forming an irradiation tunnel and fitted with UV lamps and optionally HPA lamps for tanning the face of a user. The solarium can be used in the vertical and horizontal positions, alternatively. Document DE 43 14 679 A1 relates to a device disclosed to be suitable to improve the insufficient tanning of the shoulders usually achieved on a tanning bed and, thus, is expected to achieve a tanning of the user's shoulder which better resembles to natural tanning under the sun. The device has a reflector integrated in a head pillow, which reflector provides uniform UV radiation to the user's whole shoulder skin area by reflecting the UV light emitted by the high pressure UV light sources tanning the user's face. Alternatively, low-pressure or high-pressure lamps arranged in the pillow may emit tanning UV radiation directed to the user's shoulders.

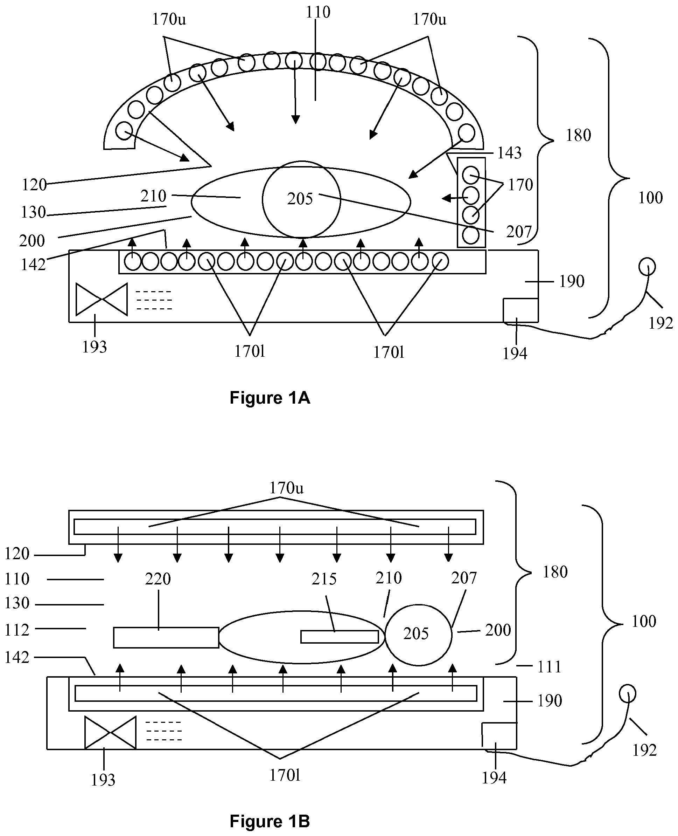

One of the disadvantages observed by many users was the fact that not all areas of the user's body could receive that type or amount of radiation to which other areas of the user's body user is exposed. Without restriction, the user's scalp, shoulders, the areas between the user's arms and the upper part of the body and the areas between the user's legs. This fact is, exemplarily, shown by Figures 1A and 1B: The user 200 lying in (although not shown: similarly: standing within) the irradiation/exposition tunnel 110 of the irradiating device 100 can receive radiation (exemplified by the dotted arrows) irradiated by the light sources 170 on his front and back sides, while certain parts of the user's body (shown: the scalp 207 and the shoulders 210) can hardly be exposed to such radiation. In addition, a tanning device as the one disclosed in the document DE 43 14 678 A1 addressed above makes the tanning bed structure more complicated. Other systems providing additional radiation sources to irradiate user's scalp or shoulders are described in documents DE 195 02 983 A1, DE 87 01 889 U1, US 2004/088028 A1 or DE 295 16 572 U1.

The above disadvantages should be overcome. Hence, it was an object of the present invention that an irradiating device and a non-therapeutic irradiation method be provided which allow irradiating such areas of the user's skin (as, for example, the shoulders) with beneficial radiation.

### Description of the Invention

Hence, the invention relates to a device for an application of directed actinic radiation to a subject, said device comprising:
- an exposition tunnel being capable of surrounding said subject to be exposed to said actinic radiation, said exposition tunnel being formed of one semi-cylinder barrel-shape first surface made of a material substantially permeable to said actinic radiation to be applied to the subject, and of at least one second surface made of said material substantially permeable to said actinic radiation to be applied to the subject, said at least one second surface being capable of complementing the first surface in forming said exposition tunnel;
- the surfaces of said exposition tunnel separating an inner space where said subject is exposed to said actinic radiation, from outer spaces, where a plurality of radiation sources capable of emitting actinic radiation through said surfaces are mounted;
- said outer spaces being housed in at least first and second parts of a chassis of the device and comprise means for fixedly holding, and electrically and electronically operating, said plurality of radiation sources; means providing said at least first and second parts of the chassis with electric power; means capable of ventilating, heating and/or cooling the device; and means for the operation of the device, including a processing unit;
- said first and second parts of the chassis of the device being configured to complement each other in forming the outer shape of the device by being configured to hingedly move away from, or towards, each other;
said device further comprising
at least one further outer space separated from said inner space by not any of said radiation-permeable first and second surfaces and located at either one or both of the longitudinal ends of the exposition tunnel, said at least one further outer space housing a plurality of additional radiation sources configured to emit radiation towards the subject in a direction forming an acute angle with the longitudinal axis A - A of the device, and at least one cooling means for the plurality of additional radiation sources, wherein the additional radiation sources comprise LEDs, and said device further comprises at least one narrow space light appliance cooling system comprising heat pipes for cooling the plurality of additional radiation sources.

Further preferred embodiments of the device are claimed in dependent claims 2 et seq..

The invention also relates to a non-therapeutical method of operating a device applying a directed actinic radiation to a subject, said method comprising the steps of
- providing a device as described in detail below in the detailed description of the invention; and
- operating at least one of the device's additional actinic light radiation-emitting light sources by allowing at least one of them to emit directed actinic radiation towards a subject being accommodated on or in said device in a direction forming an acute angle with the longitudinal axis A - A of the device.

Preferred embodiments of the method are claimed in the dependent claims 12 et seq..

### Brief Description of the Figures

The present invention is now described in detail by referring to the Figures, wherein:
Figures 1A and 1B show a radiation application device of the prior art as head (Figure 1A) and side (Figure 1B) elevational views, where not all areas of the user's body (e. g. the scalp and the shoulders) could receive the radiation to which the user is exposed;

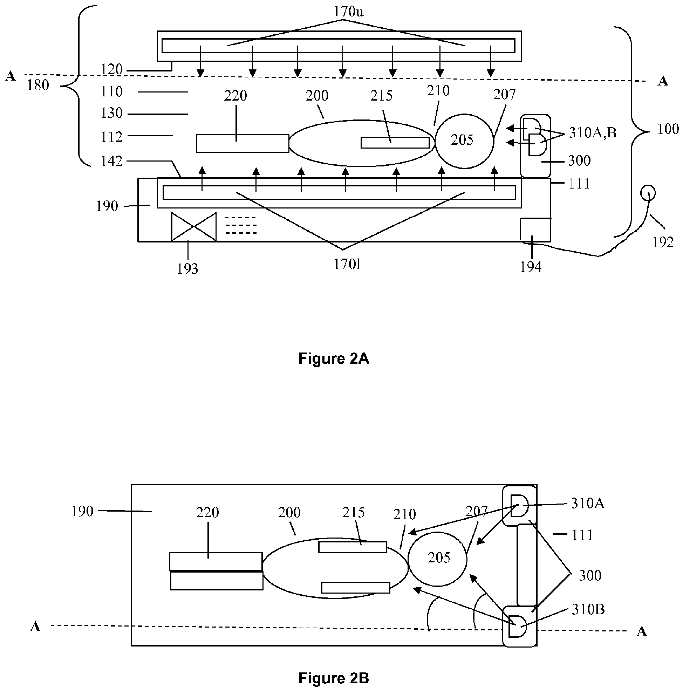

Figure 2A shows a side elevational view of the device of the present invention, similar to the one of Figure 1B (but showing a device of the present invention), which device is shown to be configured to irradiate the user with radiation, while being reclined in a horizontal exposition tunnel, by means of irradiating light sources being accommodated in a lower part of the exposition tunnel configured to irradiate the user's back and in a hingably connected canopy configured to irradiate the user's face and thorax; as well as by means of additional radiation sources configured to emit radiation towards the subject - exemplarily - from the head side longitudinal end of the exposition tunnel in a direction forming an acute angle with the longitudinal axis A of the device;
Figure 2B shows a top elevational view of the device of the present invention, similar to Figure 2A;
Figure 2C shows a perspective total view of the device shown in Figures 2A/2B;
Figure 3 shows a device of the present invention configured to irradiate the user with radiation while standing in a vertical exposition tunnel (or cylinder) by means of irradiating light sources arranged around the user;
Figure 4 shows that part of the device of the present invention which corresponds to the further outer space 300 which is configured to house additional radiation sources configured to irradiate actinic radiation - exemplarily - from the head side longitudinal end of the exposition tunnel towards the subject/user, together with its optional restricted or confined space cooling system;
Figure 5 shows a partial view of the additional radiation source housing 300 of Figure 4, specifically its LED array 350 and the additional array for accommodating high pressure lamps or - alternatively - low pressure lamps;
Figures 6A and 6B show a front view and a back view of a reflector array 360 suitable for the operation with an LED array 350 for collimating the LED light when emitted to the subject 200; and
Figures 7A to 7D show a restricted/confined space cooling system operable with the radiation application device 100 of the present invention.

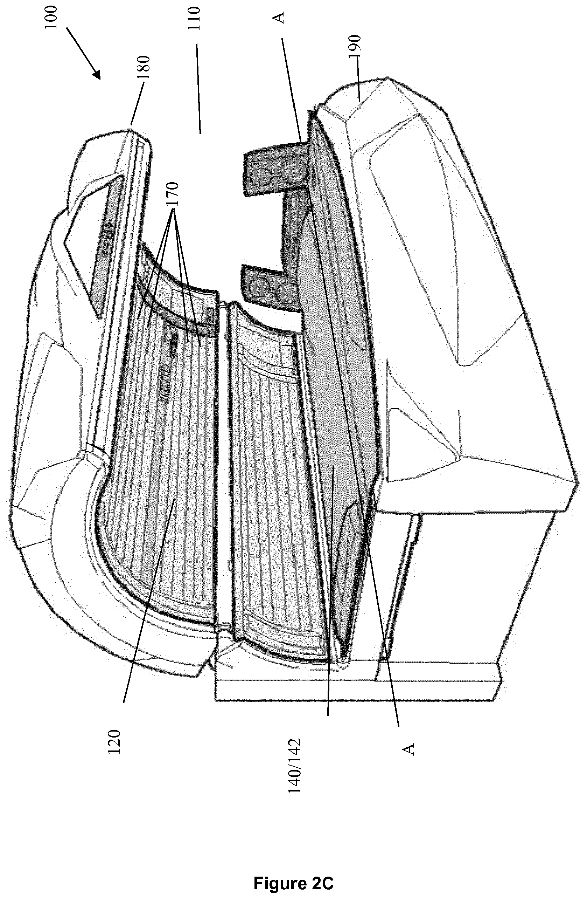

Reference is now made, for a detailed description of the invention, as broadly defined, and of its particular, in part preferred, embodiments, to the above Figures. In the following description, a reference to preferred embodiments, either in the description or in the Figures, should not be construed as a restriction of the invention to the preferred embodiments. Preferred embodiments, even if described in the description as such or shown in one or more than one of the Figures, serve only the purpose of exemplarily demonstrate the invention for a better understanding thereof. The scope of the invention can be derived by a skilled person from the claims. Moreover, the present invention is described primarily, but not exclusively, with regard to the radiation application device of the present invention shown in Figure 2C, i. e. with regard to the device wherein a subject/user is accommodated in a reclined position. However, the invention is not restricted to the embodiments of a device having its longitudinal axis arranged horizontally.

### Definitions

The terms "comprise", "comprises" or "comprising" as used in the present specification and claims, for example in claim 1 or in claim 10, has/have the meaning that the device of the invention may comprise (i) one means or may comprise (ii) two or more means as mentioned in, for example, claim 1, or that (iii) further components, means etc. (more specifically defined below) may also be comprised by the device. In a similar manner, the terms "comprise", "comprises" or "comprising" has/have the meaning that the method of the invention may comprise (i) one step or may comprise (ii) two or more steps as mentioned in, for example, claim 10, or that (iii) further steps etc. (more specifically defined below) may also be comprised by the method.

The terms "comprise" , "comprises" or "comprising" as used in the present specification and claims may, however, also include cases where the device/method of the invention mainly consists of (i) at least one means/step or mainly consists of (ii) two or more means (or steps) mentioned, for example, in claim 1 / claim 10, optionally together with any necessary component or means or step a skilled person may include into such a device / method in order to achieve the object of the invention, or may even include cases where the device / method of the invention exclusively consists of (i) at least means or step or exclusively consists of (ii) two or more means or steps, optionally, but not necessarily, together with any necessary component, means, step etc. a skilled person may include into such a device / method in order to achieve the object of the invention. Particularly in the latter case where the terms "comprise" , "comprises" or "comprising" as used in the present specification and claims may have the meaning of an "exclusively consisting of", subclaims of the present application may claim, and corresponding parts of the specification may describe, further preferred embodiments, which are characterized by additional specified features which, in combination with the features of the independent claim and corresponding parts of the description, are summarized to belong to the invention as described in its broadest scope claimed.

In other words: The terms "comprise" or "comprises" or "comprising" may have the meaning of describing a non-exhaustive enumeration of elements / steps or, alternatively, may have the meaning of describing an exhaustive enumeration of elements / steps, in the latter case without excluding further preferred embodiments being characterized by additional features.

Hence, by the terms "comprises", "comprise" or "comprising", a non-exhaustive enumeration and an exhaustive enumeration of elements or features or method steps is disclosed in the present application and claimed in the claims 1 to 12.

The features according to the present specification described in connection to the preferred embodiments may each be realized as single features or may be realized in combination with one further feature or together with few other features or with several other features or with all features described in the present specification and claimed in the claims. All these combinations of two or more features are coved by the present invention as claimed.

### Detailed Description of the Embodiments

Reference is now made to Figures 2A to 2C showing a preferred embodiment of a radiation application device 100 of the present invention.

Such a device 100 as shown in Figures 2A to 2C serves an application, to a subject 200, of directed actinic radiation.

The term "actinic radiation", as used in the present specification and claims, is understood to mean light or (broader) radiation of the whole electromagnetic spectrum (see the definition given in "Römpp Chemie-Lexikon", Thieme Publishers, Stuttgart, Germany), which has a photochemical (including a photobiochemical) effect and includes, as the case may be, light/radiation of natural or artificial origin. In the claims and specification, "actinic light" or "actinic radiation" is mainly, but not restrictively, used for light or radiation having an artificial origin, e. g. light/radiation emitted by light sources in a radiation application device 100 of the present invention. The term "directed actinic radiation", as used in the present specification and claims, has the meaning of actinic radiation which is irradiated with a preferred, if not even a more or less exclusive, focus to a target which is, in accordance with the present invention, a subject, more preferably a human subject or human user, i. e. a human being 200.

In one preferred embodiment of the device 100 of the invention, which may be realized separately or together with one or two or several or all other features of the invention, said actinic radiation may be broad wavelength range actinic radiation the wavelength or wavelength range of which is not at all restricted in the present invention. Alternatively, albeit also preferred, said actinic radiation may be narrow wavelength range actinic radiation or even actinic radiation of a very specific wavelength or several specific wavelengths. This is known to a skilled person, who may select the wavelength or wavelengths or wavelength ranges or bands to be applied in accordance with the requirements of a specific case.

Particular examples of actinic radiation having useable wavelengths in the frame of the present invention are: actinic radiation having a wavelength λ in the range of from 280 to 315 nm (UV-B radiation) and/or of actinic radiation having a wavelength λ in the range of from 315 to 400 nm (UV-A radiation) for tanning the human body for the activation of melanin generation and melanin conversion into the dark (brown) form of the pigment naturally occurring in the human skin, which tanning may serve purposes in the wellness and/or cosmetic and/or medical field(s); actinic radiation in the form of short wavelength UV-B radiation in the wavelength range of λ = 297 nm ± 10 nm for promoting Vitamin D biosynthesis from Vitamin D precursors in the human skin; or actinic radiation having wavelengths λ in the visible and near IR (infrared) range (400 or, even better > 550 nm, to 850 nm), which actinic radiation, when applied to the human skin, is capable of activating the biosynthesis of beneficial compounds for the skin's nourishing and rejuvenation and regeneration, as for example collagen, elastin, keratin and hyaluronic acid. These examples, however, should not be construed to restrict the invention.

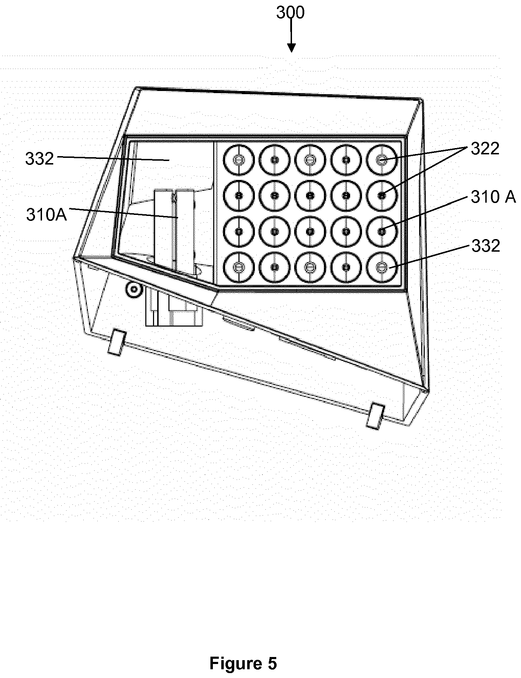

The term "directed" in combination with "actinic radiation", as used in the present specification and claims, is understood to mean actinic radiation which is emitted by an actinic radiation-emitting light source and which has a direction originating from a light source and arriving at, or impinging on, a certain target, e. g. a (general) surface A or a (specific) body surface of a subject/user 200. This is shown in Figures 1A and 1B for the prior art and in Figures 2A and 2B for the present invention. As an example, Figures 2A, 2B, 4 and 5 show light sources with reflectors and emitting actinic radiation. In the frame of the present invention, the target preferably is a subject, particularly a human being. Directed radiation may be emitted from light sources provided with shutters and/or reflectors and/or filters and/or other directing means or combinations thereof.

The term "actinic light-emitting light source" or, simply "light source", as used in the present specification and claims, is understood to mean means emitting light/radiation of a certain wavelength or of a more or less broad wavelength band. In accordance with the invention, any such light-emitting means may be designated to be an actinic light-emitting light source. A skilled person knows a great number of different light sources in the frame of the above definition. Exemplary embodiments of such actinic light-emitting light sources are gas discharge low pressure light emission lamps (or tubes) (also: low pressure fluorescent lamps) or high-pressure light emission lamps (or radiators), which usually emit light of relatively broad wavelength bands, and of which various types are well-known to a skilled person and need no more detailed description here. The term "actinic light-emitting light source" also includes LED's which usually emit light of very specific (and usually narrow) wavelength bands. All such actinic radiation-emitting light sources may be used in the present invention, as a skilled person will easily learn from the subsequent specification and from the claims.

The term "subject", as used in the present specification and claims, is understood to mean any subject to which actinic radiation can be applied in a directed manner. A skilled person knows a number of subjects (including apparatus) to which actinic radiation may be applied. In preferred embodiments of the invention, the subject is a human being, and the terms "human", "human being" and "subject" are used synonymously in the present application.

The terms "application" and "apply" and "applying", as used in the present specification and claims, are understood to mean, when combined with "actinic radiation" or (more generally) "radiation", the step of irradiating actinic radiation from an actinic radiation-emitting light source to a subject with the aim of exerting a certain photochemical or photobiochemical effect.

In accordance with the present invention, the device 100 for an application of directed actinic radiation to a subject 200 comprises several elements which are explained in detail below.

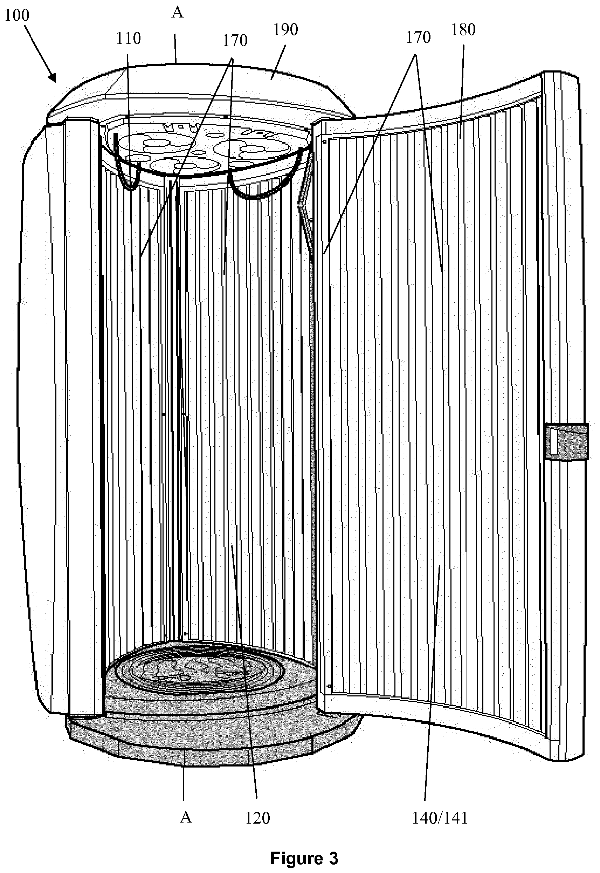

Such a radiation application device 100 may be a well-known device with respect to the majority of its elements and is described in detail generally and in its preferred embodiments, by referring to Figures 2A, 2B, 2C and 3, and in particular to Figures 2A, 2B and 2C, as follows:

The device 100 for an application of directed actinic radiation to a subject 200 comprises, as its central element, a so-called "exposition tunnel" 110. The term "exposition tunnel", as used in the present specification and claims, is understood to mean a tunnel-shaped space 110 wherein a subject 200 can be received, i. e. which exposition tunnel 110 is capable of surrounding said subject 200, and wherein said subject 200, when received, may be exposed to directed actinic radiation. The size and shape of such an exposition tunnel 110 is not restricted and may be selected by a skilled person in accordance with the requirements of the specific case. In any case, the exposition tunnel 110 must have at least a size, i. e. a longitudinal length and a width and a height perpendicular to the longitudinal length, such that the subject 200 can be surrounded generously by the exposition tunnel 100 to be reclined or to stand therein comfortably.

A skilled person knows from the general art published in this technical field the size and shape of an exposition tunnel 110 capable of surrounding a subject for the application of actinic radiation. In preferred embodiments of the invention (as in the prior art), the exposition tunnel may have a size suitable to house the subject in a convenient (lying, i. e. recumbent, or standing, i. e. erect) position. Hence, the size may be from 200 to 220 cm in length, 90 to 120 cm in width/height around said subject, without restricting the invention to such a size. In further preferred embodiments of the invention (not restricting the invention), the shape of an exposition tunnel 110 is an (at least approximate) cylinder shape.

In one preferred embodiment of the radiation application device 100 of the invention, which may be realized separately or together with one or two or several or all other features of the invention, said exposition tunnel 110 has a longitudinal axis A - A, i. e. an axis identical to the axis of a cylinder shape, which is arranged horizontally, i. e. parallel to the ground on which the device 100 is positioned when in operation. Devices 100 comprising said exposition tunnel 110 having the axis horizontally arranged are usual exposition "beds" receiving the subject 200 in a recumbent position. This is evaluated by most users of the device 100 to be very convenient and relaxing.

Moreover, actinic radiation may advantageously also be irradiated onto parts of the subject's / user's body in accordance with the present invention which are not at all, or only to a minor extent, targeted by radiation emitted by the radiation sources 170 of the device 100 of the invention.

An example of such a device 100 is shown in Figures 2A, 2B and 2C. As seen exemplarily from Figure 2A, the device 100 comprises a first part 180 of a chassis and a second part 190 of a chassis, and the first (in operation of the device 100: the upper) part 180 can be pivoted away from the second (in operation: the lower) part 190 along an axis parallel to the longitudinal axis A - A of the exposition tunnel 110, so as to allow the subject / person 200 entering into the device 100, i. e. onto the exposition bed within the exposition tunnel 110, in an easy and convenient manner.

In one alternative preferred embodiment of the device 100 of the invention, said exposition tunnel 110 has a longitudinal axis, i. e. an axis identical to the axis of a cylinder shape, which is arranged vertically, i. e. perpendicular to the ground on which the device 100 is positioned when in operation. Devices 100 comprising said exposition tunnel 110 having the axis vertically arranged are exposition "cabins" receiving the subject 200 in an erect or standing position. This is evaluated by most users of the device 100 to be very dynamic while in move. Moreover, such a device 100 obviously is very space-saving.

Moreover, actinic radiation may advantageously also be irradiated onto parts of the subject's / user's body in accordance with the present invention which are not at all, or only to a minor extent, targeted by radiation emitted by the radiation sources 170 of the device 100 of the prior art as shown in Figures 1A and 1B. An example of such a device 100 is shown in Figure 3. As seen exemplarily from Figure 3, the device 100 comprises a first (or front) part 180 and a second (or rear) part 190 of a chassis, and the first (in operation of the device 100: the front) part 180 can be pivoted away, in a manner similar to a door, from the second (in operation: the rear) part 190 along an axis parallel to the longitudinal axis A - A of the exposition tunnel, so as to allow the subject / person 200 entering into the device 100, i. e. into the exposition cabin within the exposition tunnel 110, in an easy and convenient manner.

Depending upon the type of device 100 (i. e. accommodating the subject in a recumbent or erect position), said exposition tunnel 110 is formed of one semi-cylinder barrel-shape first surface 120 and of at least one second surface 140, wherein said at least one second surface 140 is capable of complementing the first surface 120 in forming said exposition tunnel 110. This means that, in specific embodiments of the device 100 of the invention, an exposition tunnel 110 may be defined in shape and size by said first and second surfaces 120, 140 completely.

In more preferred embodiments of the radiation application device 100 of the invention, which may be realized separately or together with one or two or several or all other features of the invention, said exposition tunnel 110 is formed of said one semi-cylinder barrel-shape surface 120 and of at least one substantially flat surface 142. Preferably, this is an embodiment of the device 100 having the shape of an exposition "bed" on which the subject may be exposed to directed actinic radiation in a recumbent position: The subject 200 is positioned on said substantially flat surface 142 which, in this embodiment, is in a horizontal orientation. In such an embodiment, said one semi-cylinder barrel-shape surface 120 is arranged above said substantially flat surface 142, usually capable of being moved away from said flat surface 142 by pivoting said semi-cylinder barrel-shape surface 120 around an axis parallel to one side edge of said flat surface 142 so as to "open" the exposition tunnel 110 for an easy entry of the subject 200 for actinic radiation exposure.

The term "substantially flat" in connection to the subject's lying surface 142, as used in the present specification and claims, is understood to mean a surface which extends into a horizontal plane substantially without recesses and elevations. As is usual, however, in the present field of the art, such a lying surface for the subject 100 to be exposed to actinic radiation may include recesses for a positioning of the subject's head 205, back, arms 215 or legs 220, without substantially departing from the overall flatness.

In similarly preferred alternative embodiments of the device 100 of the invention, which may be realized separately or together with one or two or several or all other features of the invention, said exposition tunnel 110 is formed of said one semi-cylinder barrel-shape surface 120 and at least of one further, or second, semi-cylinder barrel-shape surface 141. Preferably, this is an embodiment of the device 100 having the shape of an exposition "cabin" within which the subject 200 may be exposed to directed actinic radiation in an erect position: The subject 200 is positioned between said two semi-cylinder barrel-shape surfaces 120, 141 which, in this embodiment, are in a vertical arrangement. In such an embodiment, said one semi-cylinder barrel-shape surface 120 is arranged side by side with said second semi-cylinder barrel-shape surface 141 so as to form a full cylinder. One part of said cylinder usually is capable of being moved away from said second semi-cylinder barrel-shape surface 141 by pivoting said semi-cylinder barrel-shape surface 120 around at least one axis parallel to one vertical edge of said second semi-cylinder barrel-shape surface 141 so as to "open" the exposition tunnel 110 for an easy entry of the subject 200 for actinic radiation exposure. The subject 200 stands substantially in the center of a cylinder-shape space formed by the two semi-cylinder barrel-shape surfaces 120, 141 and, while being exposed to actinic radiation, optionally, is turning around his/her own vertical axis.

In accordance with the invention, the semi-cylinder barrel-shape first surface 120, and the at least one second surface 140 as well, are made of a material substantially permeable to said actinic radiation to be applied to the subject 200. The term "material substantially permeable to said actinic radiation" as used in the present specification and claims, is understood to mean that the material of the surfaces is of such a nature that at least that portion of the actinic radiation emitted by the light sources for having the desired photochemical effect or photobiochemical effect can pass said walls without that substantial amounts thereof are absorbed by the surface material. Moreover, the material selected is required to have a sufficient strength and rigidity to bear the user lying thereon. A skilled person in this technical field knows such materials and may select them from a number of materials in accordance with the requirements of a specific case. In particularly preferred embodiments of the invention, the surfaces 120, 140 are made from a material selected from the group of actinic light-permeable polymeric materials, and acrylic polymers are particularly preferred, as also in the prior art. Also, other known prior art materials capable of allowing a passage of substantially all actinic radiation directed thereto may be used alone or in combination.

In an even more preferred embodiment of the invention, an additional surface 143, similar to the semi-cylinder barrel-shape first surface 120 and the at least one second surface 140, is also made of a material substantially permeable to said actinic radiation to be applied to the subject 200.

In accordance with the invention, the device 100 for an application of directed actinic radiation to a subject 200 is shaped in a way that the surfaces 120, 140 and, optionally also any additional surface 143, of the exposition tunnel 110 separate an inner space 130, where a subject 200 to be exposed to actinic radiation is surrounded by said surfaces and is exposed to said actinic radiation, from outer spaces 150, 160 where light sources 170 capable of emitting actinic radiation through said surfaces 120, 140 are mounted in such a way that said light sources can emit said actinic radiation through said surfaces 120, 140 towards the subject 200. Such outer spaces 150, 160 may have any shape a skilled person may consider suitable for the purpose of, *inter alia,* accommodating said light sources 170, without that the invention is restricted in any way. Particularly, the shape of said outer spaces 150, 160 may depend on the type of device 100 used.

In preferred embodiments of the device 100 of the invention, which may be realized separately or together with one or two or several or all other features of the invention, if said exposition tunnel 110 has a longitudinal axis A - A arranged horizontally, as shown in Figures 2A, 2B and 2C, upper light sources 170u capable of emitting actinic radiation through said surface 120 downwards to the subject 200 lying in the recumbent position on said substantially flat surface 142, and lower light sources 1701 capable of emitting actinic radiation through said surface 140, 141, 142 upwards to the subject 200 lying in the recumbent position are mounted above said surface 120 in said outer space 150 and below said surface 140, 141, 142 in said outer space 160. In this embodiment, the outer spaces 150, 160 are upper and lower spaces and are used, *inter alia,* for an accommodation of said upper and lower light sources 170u, 1701. The outer spaces 150, 160 may be used in accordance with the invention for other purposes, too, which are described below in further detail.

In alternative preferred embodiments of the device 100 of the invention, which may be realized separately or together with one or two or several or all other features of the invention, if said exposition tunnel 110 has a longitudinal axis A - A arranged vertically (as shown in Figure 3), front side light sources 170fs capable of emitting actinic radiation through said surface 120 backwards to the subject 200 standing in an erect position in said inner space 130 surrounded by said surfaces 120, 140, and back side light sources 170bs capable of emitting actinic radiation through said surface 140 towards the standing subject 200 are mounted on the outer sides of said surfaces 120, 140 in said outer spaces 150, 160. In this alternative embodiment, the outer spaces 150, 160 are front side outer spaces and back side outer spaces, which are used, *inter alia,* for an accommodation of said front side and back side light sources 170fs, 170bs. The outer spaces 150, 160 may be used in accordance with the invention for other purposes, too, which are described below in further detail.

In accordance with the invention, said outer spaces 150, 160 are housed in at least first and second parts of a chassis 180, 190 of the device 100 and comprise means 181, 191 for fixedly holding, and electrically and electronically operating, said light sources 170 and/or means 182, 192, providing said at least first and second parts of the chassis 180, 190, and the components therein, for example said light sources 170 emitting actinic radiation to the subject 200, with electric power and/or means 183, 193 capable of ventilating, heating and/or cooling the device 100; and means 184, 194 for the further operation of the device 100, as for example control means and/or at least one processing unit for controlling the operation of the device 100 and/or for allowing external control means to be connected. Further means as known to a skilled person from similar devices of the prior art may also be mounted, singly or in combination, to the outer spaces 150, 160 of the first and second parts 180, 190 of the chassis, as for example handles for opening and closing the movable chassis parts 180, emergency switches capable of stopping the operation of the device 100 in cases of emergency, nozzles (and their operation channels and pumps) for allowing an access of fresh, cooling or warming air or of liquid sprays to the exposition tunnel 130, a central processing unit 194 capable of operating the device 100, actinic radiation sensors, a skin colour/skin status sensor for determining the subject's/user's skin condition as far as relevant for an actinic radiation exposure, to enumerate only few examples.

In preferred embodiments of the invention, which may be realized separately or together with one or two or several or all other features of the invention, the subject 200 is a human being 200 to be exposed to said directed actinic radiation. Human beings 200 to be exposed to said directed actinic radiation are sometimes also referred to, in this specification, as "users" of the device 100.

As already described above in detail, in accordance with the present invention, the first and second parts of the chassis 180, 190 of the device 100 are capable of complementing each other in forming the outer shape of the device 100 by hingedly moving away from, or towards, each other. As seen exemplarily from Figures 2A and 2C, the device 100 comprises a first part 180 and a second part 190 of a chassis, and the first (in operation of the device 100: the upper) part 180 can be pivoted away from the second (in operation: the lower) part 190 along an axis parallel to the longitudinal axis A - A of the exposition tunnel 110, so as to allow entering into the device 100, i. e. onto the exposition bed within the exposition tunnel 110, in an easy and convenient manner.

In accordance with the invention and as shown in Figures 2A and 2B, the device 100 further comprises at least one further outer space 300 separated from said inner space 130 by not any of said radiation-permeable first and second surfaces 120, 140 and located at either one or both of the longitudinal ends, i. e. head side end 111, in a longitudinal direction, of the exposition tunnel 110 and foot side end 112, in a longitudinal direction of the exposition tunnel 110. In accordance with the invention, said at least one further outer space 300 houses a plurality of additional radiation sources 310A, 310B capable of emitting radiation towards the subject 200 in a direction forming an acute angle with the longitudinal direction of the device 100. There may be one further outer space 300, or there may be two (or even more, e. g. three or four) further outer spaces 300 located at one or at two of the longitudinal ends 111, 112 of the exposition tunnel 110, suitable and configured to accommodate a plurality of additional radiation sources 310A, 310B and capable of emitting radiation towards the subject 200 in a direction forming an acute angle with the longitudinal direction of the device 100. A selection of the number of further outer spaces 300 may be made by a skilled person on the basis of the requirements of a single case.

In preferred embodiments of the invention, which may be realized separately or together with one or two or several or all other features of the invention, said at least one further outer space 300 is one further outer space 300 located at the head-side longitudinal end 111 of the exposition tunnel 110, said one further outer space 300 housing a plurality of additional radiation sources 310A, 310B capable of emitting radiation towards, for example and without restriction, the subject's scalp 207 and shoulders 210. This embodiment of the invention is considered to be particularly advantageous, because the object of the invention can be achieved easily: Areas of the user's body not targeted by irradiated light on usual irradiating devices 100 can receive radiation as desired.

In further preferred embodiments of the invention, which may be realized separately or together with one or two or several or all other features of the invention, the device 100 of the invention may be capable of irradiating radiation of different types onto a user 300:

In one preferred embodiment, the device 100 is a tanning device irradiating tanning radiation onto a user 200, e. g. radiation having an intensity suitable for tanning the user's body in the wavelength ranges of from 280 to 315 nm (UV-B radiation) and/or radiation having a wavelength of from 315 to 400 nm (UV-A radiation). Conventional tanning devices did not effect, in a technically simple manner, tanning of the user's shoulders 210 or scalp 207, for example, and a uniform tan on the user's skin could hardly be achieved. In accordance with the present invention, also those parts of the user's body usually not targeted by tanning UV radiation (e. g. the shoulders 210) can be tanned reliably, thereby achieving a uniform tan of the user's whole body.

In another preferred embodiment, the device 100 is a device irradiating onto the user's body visible red light and/or near infrared light of wavelengths in the range of from 400, or better from > 550 nm, to 850 nm, thereby activating the synthesis of beneficial compounds for the human skin's nourishing and rejuvenation and regeneration, e. g. collagen, elastin, keratin, and hyaluronic acid.

For these and other purposes, in preferred embodiments of the invention, which may be realized separately or together with one or two or several or all other features of the invention, the plurality of additional radiation sources 310A, 310B is selected from the group consisting of high-pressure lamps, medium pressure lamps, gas discharge low-pressure lamps, LEDs and combinations thereof. Even more preferably, the plurality of additional radiation sources 310A, 310B is selected from the group consisting of high-pressure lamps, LEDs and combinations thereof.

While high pressure lamps or low pressure lamps may be used, in accordance with the invention, as single radiation-emitting sources or as pairs of two or groups of three or more of them in the device 100 of the present invention, LEDs may preferably be used as a plurality of LEDs arranged in arrays of, for example, 20 or 30 or even less of them or even more of them, fixed and electrically connected and electronically controlled on one board.

In further preferred embodiments of the invention, which may be realized separately or together with one or two or several or all other features of the invention, for the example of an irradiating device 100 suitable for tanning the user's body, several combinations of additional radiation-emitting radiation sources 310A, 310B were developed and found to be suitable, without being restricted to these preferred embodiments.

While the present invention proposes that, for targeting tanning radiation to parts of the user's body which, when using prior art tanning devices, do not receive tanning radiation at all or receive only amounts of tanning radiation evaluated to be insufficient for the desired tanning result, LEDs be used as additional radiation sources 310A, 310B, a good shoulder, scalp etc. tanning may be achieved also, in accordance with the present invention, by irradiating the subject's/user's shoulder (as an exemplary embodiment of the invention) or scalp by means of low pressure fluorescent lamps or high-pressure discharge lamps or LEDs emitting the suitable wavelength (range) radiation alone or in each conceivable combination. Particularly suitable are combinations of LEDs with low pressure fluorescent lamps or combinations of LEDs with high pressure discharge lamps, all of them emitting tanning UV radiation of the relevant UV-A and UV-B ranges. Of course, a skilled person is not restricted by the present invention to select radiation sources emitting such relevant UV-A and UV-B ranges in accordance with the requirements of a single case. In further preferred embodiments, such UV radiation may also be combined with radiation of the visible and/or IR (infrared) wavelengths ranges for specific purposes, as a skilled person will recognize, without being bound by any restrictions imposed by the present invention.

In other words: In further preferred embodiments of the invention, which may be realized separately or together with one or two or several or all other features of the invention, for the example of an irradiating device 100 suitable for tanning the user's body, the usual low pressure (fluorescent) lamps may be used as the light/actinic radiation sources 170. Moreover, it is possible (as another preferred embodiment of the invention) that medium-pressure or high pressure (discharge) lamps be used in addition (i. e. combined with low pressure lamps) for specific purposes: As is known to a skilled person, in tanning devices as the devices 100 of the present invention, high pressure lamps (radiators) may be employed, e. g. for tanning specific parts of the user's body, e. g. the user's face. In further preferred devices 100 of the invention, LEDs emitting the appropriate radiation of tanning light wavelengths may be used, alone (i. e. instead of) or in combination with other radiation-emitting light sources 170, e. g. low pressure (fluorescent) lamps and/or medium pressure or high pressure (discharge) lamps. In particularly preferred embodiments of devices 100 of the invention, the light/actinic radiation-emitting light sources 170 are low pressure (fluorescent) lamps and/or high pressure (discharge) lamps, and the additional light/radiation sources 310A and 310B are selected from either combinations of LEDs and low pressure lamps and/or high pressure lamps and LEDs or from LEDs, exclusively. A skilled person may select the suitable light/radiation sources 170 and/or 310A/310B, and combinations thereof, of the devices 100 of the present invention on the basis of the requirements of a single case, without being bound by any restrictions.

In further preferred embodiments of the invention, which may be realized separately or together with one or two or several or all other features of the invention, the device 100 may be provided with further lamps / light sources in order to achieve a number of specific objects, as for example improving the tanning effect of tanning light sources and/or stimulating a synthesis of collagen, keratin or other biochemically beneficial compounds. Such stimulation may preferably be initiated (without restriction) by emitting, onto the user's body or parts thereof, light / radiation in wavelength ranges from 400 nm (visible wavelengths) to 850 nm (near infrared wavelengths) or in partial ranges thereof. Non-restricting examples for LEDs emitting visible light / radiation in specific wavelengths are shown in the following Table:

**Table non-restricting examples of suitable LED wavelength ranges/wavelengths**

| **Wavelength (range) [nm]** | **Intended Purpose** |
|---|---|
| 545 - 550 (540) | Stimulation of PPIX (Protoporphyrin IX) in a photodynamic therapy (PDT) |
| 585 - 590 (580) | Stimulation of PPIX (Protoporphyrin IX) in a photodynamic therapy (PDT) |
| 610-615 (613.5 - 623.5) | "Optimum compromise" wavelength range between stimulation wavelength H₂O molecule (606 nm) and the lower stimulation wavelength of Cytochromic C Oxidase (613.5 nm - 623.5 nm) (range 1) |
| 635 - 640 | "Optimum compromise" wavelength range between stimulation wavelength PPIX (PDT) (635 nm) and the upper stimulation wavelength of Cytochromic C Oxidase (613.5 nm - 623.5 nm) (range 1) |
| 670 - 675 | Stimulation of Cytochromic C Oxidase (667.5 nm - 683.7 nm) (range 2) |
| 750 - 755 | Stimulation of Cytochromic C Oxidase (750.7 nm - 772.3 nm) (range 3) |
| 830 - 835 | Stimulation of Cytochromic C Oxidase (812.5 nm - 846.0 nm) (range 4) |

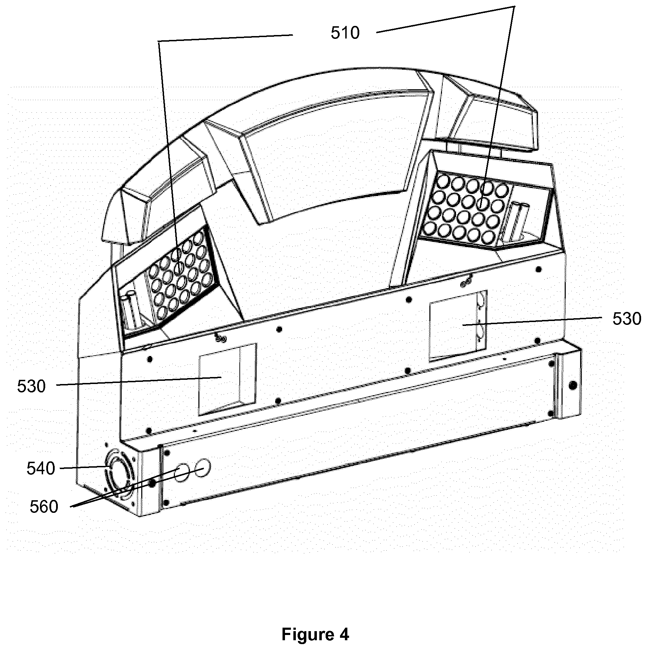

In further preferred embodiments of the invention, which may be realized separately or together with one or two or several or all other features of the invention, the device 100 may comprise further elements, for example for improving the efficacy of the light emitted by the light sources 170 and/or 310A/310B. With reference to Figures 2A, 2B, 4 and 5, it is exemplified (without restricting the invention to such embodiments) that light sources 170 and/or 310A/310B emitting actinic radiation in accordance with the invention may be provided with shutters and/or reflectors and/or filters and/or other directing means or combinations thereof. In the frame of the present invention, the target preferably is a subject, particularly a human being. In particularly preferred embodiments of the invention in cases where LEDs are employed, e. g. in cases of additional radiation-emitting light sources 310A and 310B of the invention, LED arrays comprising a larger number of LEDs (as for example, but not restricting, 16 LEDs, 20 LEDs, 24 LEDs or any other preferred number of LEDs), an "array" of reflectors may be provided. As is known to a skilled person in this technical field, the radiation emitted by each of the LEDs each provided with a reflector can be collimated, i. e. concentrated and directed to a predeterminable, relatively small area at the target (here: on the user's body or on parts thereof), thereby improving its efficacy. In particularly preferred embodiments of the invention, at least one reflector, even more preferred: one reflector per LED, is used with the aim of collimating the radiation emitted by said LEDs as the additional light/radiation sources 310A, 310B. This is shown in Figures 4, 5, 6A and 6B. Similarly, light sources other than LEDs and emitting suitable tanning radiation to the subject's/user's body may be provided with suitable means, e. g. reflectors, for collimating and/or concentrating the emitted radiation to the desired target, i. e. to the object's/user's body, especially to parts thereof, more preferably to those parts not receiving sufficient tanning radiation when using prior art tanning devices.

In further preferred embodiments of the invention, which may be realized separately or together with one or two or several or all other features of the invention, the device 100 may comprise means for cooling the light sources 170 and/or the additional light sources 310a / 310b. As is well known to a person skilled in the present field of the art, a considerable part of the energy emitted by the light sources 170 and/or by the additional light sources 310a / 310b is emitted in the form of heat radiation. Particularly high pressure "discharge" lamps as well as LEDs usually emit heat radiation in addition to the radiation of the UV, visible and/or IR radiation wavelengths which they are devised for. The emitted heat may beneficially be used for warming up either the radiation tunnel's air volume (where the subject to be irradiated is reclining or standing) or at least one part of the irradiation device 100, for example (but not restricting) the light-transparent protection layer on which the subject/user to be irradiated is reclining. On the other hand, it has to be considered that the heat emitted by the light sources must be dissipated in order not to deteriorate the light source lamps' functions and not to shorten the light source lamps' service life. Dissipating the heat developed and emitted by the light sources may be achieved by means known to a skilled person from the prior art, and no restriction is imposed to a skilled person in the present invention in this respect. Hence, suitable cooling means may be selected by a skilled person in accordance with the circumstances of a single case.

While cooling means for lamps of the radiation sources 170 of the invention, i. e. for those accommodated above/behind the semi-cylinder barrel-shape first surface 120 and emitting radiation to the subject's / user's face and belly and for those accommodated below/behind the second surface 140 (either below/behind the semi-cylinder barrel-shape second surface 141 or below/behind the substantially flat second surface 142) can be selected without any restrictions concerning the available space for their accommodation and way of operation. Hence, such cooling means may be usual fans blowing air against the radiation sources for cooling, or may be usual cooling jackets making use of cooling fluids (including liquid cooling fluids) for dissipating the excessive heat. However, such usual cooling means cannot be used in cases where the space for accommodating the radiation sources as such is restricted already, as proposed as one alternative, e. g. when using arrays of a plurality of LEDs, thereby confining the space for (further) accommodation of cooling means considerably. Hence, in further preferred embodiments of the invention, which may be realized separately or together with one or two or several or all other features of the invention, the device 100 may comprise a restricted/confined space cooling system 500 as a cooling means for dissipating the heat generated / emitted by the light sources' lamps and not required for any beneficial heating purpose. Even more preferred in accordance with the invention, and optionally realized separately or together with one or two or several or all other features of the invention, the device 100 of the invention may comprise a restricted space light appliance cooling system 500 with the aim of, and for the purpose of, cooling (and thereby dissipating the heat generated by) the plurality of additional radiation sources 310A, 310B configured to emit radiation towards the subject's / user's scalp 207 and shoulders 210. In this context, in the present specification and claims, the terms "narrow space" or "restricted space" or "confined space" are used interchangeably or synonymously and have the meaning that, due to requirements imposed by the device or its components to be cooled, only an insufficiently narrow or restricted or confined space is available for accommodating the components to be cooled together with a cooling system effecting the cooling action. Due to the insufficiency of space for accommodating the complete cooling appliance in close proximity to the components or devices to be cooled, the cooling system is splitted into a heat absorption component absorbing the heat where it is generated and a heat dissipation component dissipating the heat at a distance away from the heat generation area, and the heat is "transported" efficiently from the heat generation area to the heat dissipation area, without a requirement of spending additional energy for the heat transport.

In the present specification and claims, the terms "proximal" and "distal" are terms used to indicate, for the restricted space cooling system 500 used together with the invention, a short ("proximal") and long ("distal") distance between the component to be cooled (and the thermal interface 510 accommodated in close proximity to the component(s) to be cooled), on the one side, and the remaining components of a heat pipe arrangement, on the other side.

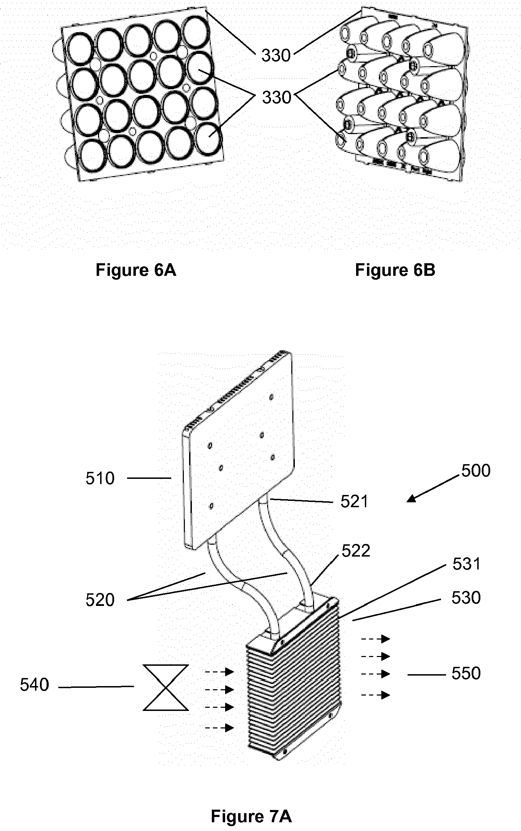

A general restricted/confined space cooling system useable as an element in, and in combination with, the radiation application device 100 of the present invention is disclosed in the parallel European Patent Application having the title "Restricted Space Cooling System and its Use" filed with the European Patent Office on the same date as the present application, which is referred to by reference and which is shown exemplarily in Figure 7A.

Such a restricted space cooling system 500 useable in the device 100 of the invention as one restricted space cooling system or as more than one (i. e. a plurality of two or of three or of even more) restricted space cooling system(s) and shown as one restricted space cooling system 500 in Figure 7A may comprise as its main components
- at least one thermal interface (510) configured to absorb heat from heat-discharging appliances closely adjoining the at least one thermal interface (510);
- at least one heat pipe (520) configured to transfer heat from the heat pipe's end (521) proximal to the thermal interface (510) to the heat pipe's end (522) distal from the thermal interface (510);
- at least one heat exchanger (530) or heat sink (530) configured to dissipate the heat released by the heat pipe distal end (522); and optionally
- at least one heat dissipation means (540) configured to force a heat-absorbing medium (550) to take up and dissipate the latent heat from the heat exchanger (530) to an environment.

In accordance with the proposal of the present invention, the above main components of a restricted space cooling system 500 may be part of the restricted space cooling system 500 as at least one of each of the components. In other embodiments of the invention, one or two or all of the components may be part of the restricted space cooling system 500 as two or three or even more of them. As an alternative to the invention described above, one of the components (e. g. - without restriction - the thermal interface 510) may be present as one thermal interface 510, while one of the other components (or even two or all other components of the restricted space cooling system 500) may be present as two or three of them, e. g. two, three or four heat pipes 520 and one or two or three heat exchangers 530 and one (or two) heat dissipation means 540. A skilled person may select, and vary, the number of each of the components of the restricted space cooling system 500 of the invention in accordance with the conditions and circumstances and requirements of a single case.

The thermal interface 510 of the restricted space cooling system 500 shown in Figure 7A is not in any way restricted, as long as it is configured to absorb heat from any heat-discharging device into close proximity to which the at least one thermal interface 510 is brought. In the context of the device 100 of the present description and claims, "close proximity" is understood to mean that, for an optimized heat conduction between a heat-generating device 310A, 310B, i. e. an additional radiation source 310A, 310B, and the thermal interface 510 of the restricted space cooling system 500 shown in Figure 7A, the jointing surfaces need to have intimate contact to each other. This requires that each of the surfaces has to be made of a material having a high coefficient of thermal conduction, preferably of a metallic material, and the materials ideally should have a fine finish and should be flat or co-planar to each other. In cases of surface discrepancies of roughness and undulation, it is recommendable using suitable interfacing materials and/or heat-conducting pads or a heat-conducting paste to achieve close fitting. Hence, in preferred embodiments of the invention, and as shown in Figure 7A, the thermal interface 510 made of a material having a high value of heat conductivity, and particularly of suitable materials as, for example, a metal material like aluminum, copper, steel and their alloys, e. g. a steel sheet, is a flat plate adapted to fit with a heat-discharging appliance from which heat is to be absorbed.

In further preferred embodiments, which may be realized with one or two or all other features of the invention and which should not be construed to restrict the invention, the at least one thermal interface 510 of the restricted space (light appliance) cooling system is at least one backing plate 321 of a fixture for lighting appliances, which discharge heat due to their characteristic energy emission spectrum. It is even more preferred that the at least one thermal interface 510 is at least one backing plate 321 of a fixture for a LED array. LED arrays are known to accommodate a plurality of LEDs on a fixture, where the LEDs are in close proximity to each other and do emit not only light, usually of at least one relatively narrow wavelength band, but do also emit considerable amount of heat (depending on their mode of operation). Due to the closely adjacent mounting of the LEDs on the array, the space for providing a cooling system for sufficient cooling is restricted/confined. If the backing plate of the LED array fixture is used as the at least one thermal interface 510, heat evolving from the LEDs' operation can be dissipated away from the LED array, thereby improving the LEDs' operation and largely extending the LEDs' durability and life of installation and life of operation.

As shown in Figure 7A, the second component of the restricted space cooling system 500 useable in the device 100 of the invention is at least one heat pipe 520. The at least one heat pipe 520 is, or one or two or three or even more heat pipes 520 are, configured to transfer heat from the at least one thermal interface 510 to at least one heat exchanger 530 via the at least one heat pipe 520, namely from the heat pipe's end 521 proximal to the thermal interface 510, which takes the heat from any heat-discharging appliance, as for example from a light appliance as a LED array, to the heat pipe's end 522 distal from the thermal interface 510 by the action of a fluid enclosed in the at least one heat pipe's inner volume. When receiving the heat, at the heat pipe's end 521 proximal to the thermal interface 510 (i. e. the heat pipe's proximal end 521) from the at least one thermal interface 510, the fluid in the phase of a liquid experiences a phase transition to become a vapor, said vapor of the fluid being generated while inducing heat taken from the at least one thermal interface 510. Said vapor moves within the at least one heat pipe 520 "down" (with respect to the temperature differential ΔT within the at least one heat pipe 520, even if such motion may be one against the direction of gravity) to the "cooler" heat pipe end distal from the at least thermal interface 510. i. e. to the heat pipe distal end 522. At the heat pipe distal end 522, there is effected a phase transition of the fluid from the vapor phase to the liquid phase, i. e. by condensation of the vapor back to the fluid's liquid phase, under latent heat release at the heat pipe distal end 522 to the at least one heat exchanger 530. The condensed liquid phase fluid then returns to the heat pipe proximal end 521 "up" (with respect to the temperature differential ΔT within the at least one heat pipe 520, even if such motion may be one with the direction of gravity) to the "heated" heat pipe end proximal to the at least thermal interface 510. i. e. to the heat pipe proximal end 521 for another heat transfer cycle.

In other words, the motion of the fluid, neither in the vapor phase nor in the liquid phase, within the at least one heat pipe's inner volume requires no external motive power except that of an applied temperature differential ΔT of a thermal loading. In even more preferred embodiments, the motion of the fluid within the at least one heat pipe's inner volume, is effected by a force selected from the group consisting of capillary action, centrifugal force and gravity and combinations thereof. This is assisted by suitable configuration of the inner volume of the at least one heat pipe 520, e. g. by a porous capillary lining applied to the inside of the heat pipes' sealed metal envelope, to indicate only one (non-restricting) example.

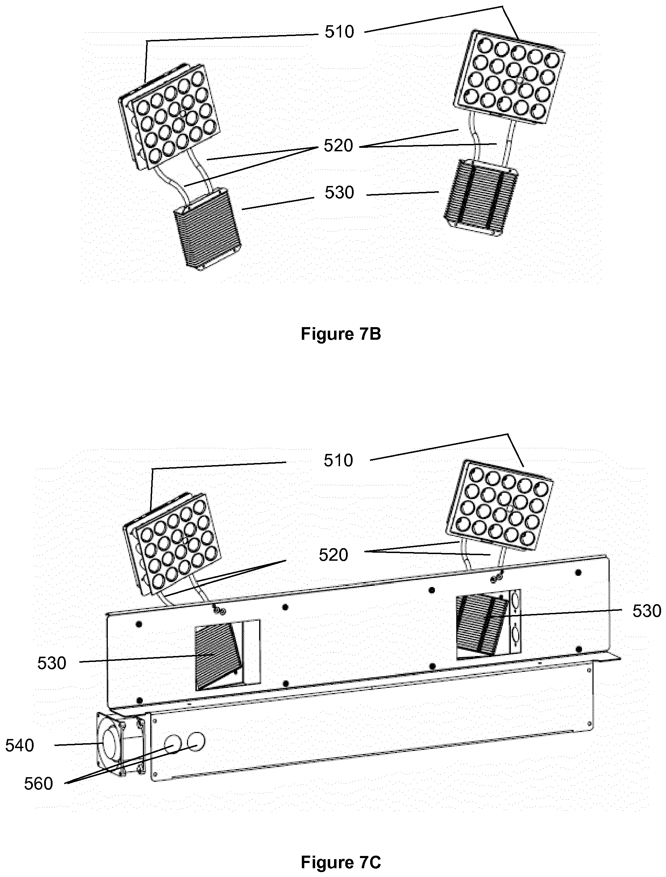

In advantageous and, hence, preferred embodiments which may be realized with one or two or all other features of the invention and which should not be construed to restrict the invention, the fluid enclosed in the at least one heat pipe's inner volume is selected from the group consisting of fluids having excellent heat-absorbing properties in a temperature range required for a specific heat pipe operation temperature and preferably is selected from the group consisting of water, ammonia, methanol, ethanol and mixtures thereof. The fluid for filling the inner volume of the at least one heat pipe 520 is not restricted for the present invention and may be selected by a skilled person in accordance with the requirements and depending upon determinants to be observed in specific cases, of which one is the temperature range within which the heat amount to be dissipated is, which temperature range should match with the temperatures of vaporization or condensation of the fluid within the inner volume of the at least one heat pipe 520. In further preferred exemplary embodiments, which may be realized with one or two or all other features of the invention and which should not be construed to restrict the invention, the restricted space cooling system 500 comprises two or more heat pipes, e. g. two, three, four, five or six heat pipes 520. Particularly preferred - without restricting the invention to this embodiment - are two heat pipes 520 per one thermal interface 510 and per one heat exchanger 530, as also shown in Figures 7B and 7C.

In advantageous and, hence, preferred embodiments which may be realized with one or two or all other features of the invention and which should not be construed to restrict the invention, the shape of the at least one heat pipe 520 or of the two or more heat pipes 520 is identical or is different. In more preferred embodiments, the shape of the two or more heat pipes 520 is straight, or the shape of the two or more heat pipes 520 is bent, or the shape of the two or more heat pipes 520 is a combination of straight and bent shape heat pipe portions.

Concerning the shape of the heat pipes 520, the present invention does not impose any restrictions to a skilled person, when selecting the shape of the heat pipes 530 in accordance with the requirements and depending upon determinants to be observed in a specific case. One of the determinants may be the space available for the narrow space cooling system 500 useable in the device 100 of the present invention. Similarly, concerning the length of the heat pipe or heat pipes 520 from its/their proximal end 521 (where the heat pipe(s) 520 emerge(s) from the thermal interface 510) to its/their distal end (where the heat pipe(s) 520 enter(s) into the heat exchanger 530), the present invention does not impose any restrictions to a skilled person, when selecting the length of the heat pipes 530 in accordance with the requirements, and depending upon determinants to be observed in a specific case. One of the determinants may be the space available for the restricted space cooling system 500 of the present invention. As non-restricting examples, the heat pipe length may be in a range of from 5 to 30 cm, preferably in a range of from 10 to 20 cm, but the length of the heat pipe(s) 520 may have values above or below the above ranges. Moreover, in certain embodiments of the invention (giving the skilled person better flexibility in selecting the heat pipe length in accordance with the requirements of a specific case), the heat pipe lengths may be different in cases where more than one heat pipe 520 is selected to be used.

As another one of the essential components of the restricted space cooling system 500 shown exemplarily in Figure 7A, the restricted space cooling system 500 comprises at least one heat exchanger 530 or heat sink 530. The at least one heat exchanger 530 is, or two or three or even more heat exchangers 530 or heat sinks 530 are, configured to dissipate the latent heat released by the condensing vapor at the heat pipe distal end 522. In the context of the present invention, there is no restriction imposed to a skilled person with respect to the heat exchanger or heat sink 530, and any known heat exchanger(s) or heat sink(s) 530 having usual characteristics of dissipating heat may be used, as long as the heat exchanger or heat sink 530 used in the narrow space cooling system 500 of the invention is/are configured to dissipate the latent heat released by the condensing vapor at the heat pipe distal end 522.

In further preferred embodiments of the invention which may be realized with one or two or all other features of the invention and which should not be construed to restrict the invention, the restricted space cooling system 500 comprises at least one heat exchanger or heat sink 530 which comprises cooling fins 531 increasing the heat exchange surface. Advantageously, the cooling fins 531 contribute to improving the heat exchange efficiency of the heat exchanger 530, and the heat dissipation, by the restricted space cooling system 500 useable in the device 100 of the invention, into the environment can be effected more efficiently.

In accordance with the present invention as shown in Figure 7A, the restricted space cooling system 500 may comprise as an additional, and optional, component as least one heat dissipation means 540 especially suited for dissipating the heat discharged from the at least one heat pipe 520 via the at least one heat exchanger or heat sink 530. The at least one heat dissipation means 540 is, and especially one or two or even three heat dissipation means are, configured to force a heat-absorbing medium 550 to take up and dissipate the latent heat from the at least one or two or three or even more heat exchanger(s) or heat sink(s) 530 to an environment of the restricted space cooling system 500, for example into the air space surrounding the restricted space cooling system 500 of the invention.

In further preferred embodiments useable in the device 100 of the invention which may be realized with one or two or all other features of the invention and which should not be construed to restrict the invention, the restricted space cooling system 500 comprises the at least one heat dissipation means 540 comprising a fan 540 blowing environmental air as the heat-absorbing medium 550 along a plurality heat exchange surfaces into the environmental gas space, as for example shown in Figure 7A. By providing a fan 540 blowing air 550 along the heat exchanger's cooling fins 531, the cooling action can be achieved effectively in a restricted space to be cooled.

As another example in accordance with a preferred embodiment useable in the device 100 of the invention, the at least one heat exchanger 530 may comprise a cooling jacket allowing passing a cooling fluid along the at least one heat exchanger, thereby forcing the cooling fluid as the heat-dissipating medium 550 to flow through and along the heat exchanger 530 and effect an efficient dissipation of the heat generated by the heat-generating appliances 10.

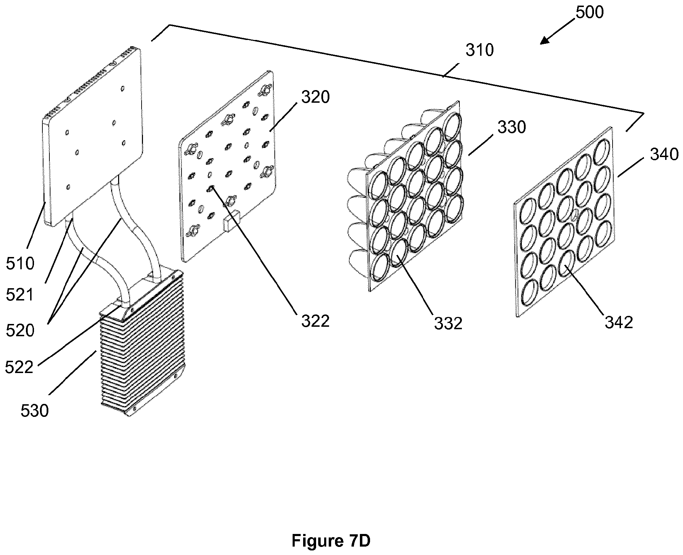

Figures 7B, 7C and 7D show other embodiments of the restricted space cooling system 500 useable in the device 100 of the present invention. As shown in Figures 7B, 7C and 7D and especially in the exploded view of Figure 7D, a narrow space cooling system 500 useable in the device 100 of the invention comprises:
- at least one light source array 320 as a part of a (heat-generating) device 10 for the application of directed actinic radiation to a subject, comprising at least one light source 310A, 310B emitting light of at least one wavelength and further discharging heat generated by its operation; said cooling system 500 further comprising:
- at least one thermal interface 510 configured to absorb heat from said heat-discharging light source array 320 closely adjoining the at least one thermal interface 510, which at least one thermal interface 510, in a further preferred embodiment of the invention, may be at least one backing plate of a fixture for a LED array 320;
- at least one heat pipe 520 configured to transfer heat generated by the at least one light source array from the heat pipe's end 521 proximal to the thermal interface 510 to the heat pipe's end 522 distal from the thermal interface 510 by phase transition of a fluid enclosed in the heat pipe's inner volume from the liquid to the vapor phase at the heat pipe proximal end 521 and motion of said vapor to the heat pipe distal end 522, where a phase transition of the fluid from the vapor to the liquid phase is effected by condensation of the vapor back to the liquid phase under latent heat release at the heat pipe distal end 522, and return of the condensed liquid phase fluid to the heat pipe proximal end 521;

- at least one heat exchanger 530 or heat sink 530 configured to dissipate the latent heat released by the condensing vapor at the heat pipe distal end 522; and optionally
- at least one heat dissipation means 540 configured to force a heat-absorbing medium 550 to take up and dissipate the latent heat from the heat exchanger 530 to an environment.

In further preferred embodiments of the invention, the restricted space cooling system 500 provided by the present invention also comprises at least one vent 560, more preferably two or more vents 560, for example (and as shown in Figure 4) two vents 560, through which the at least one heat dissipation means 540, preferably the fan 540, blows out the heat taken up from the heat exchanger 530 to an environment.

In further preferred embodiments useable in the device 100 of the invention which may be realized with one or two or all other features of the invention and which should not be construed to restrict the invention, the narrow space light appliance cooling system 500, which is especially shown in Figure 7D as an exploded view, comprises a LED appliance as the light appliance, preferably in the form on a LED array 320. As shown in Figure 7D, the LED array 320 comprises at least one LED 322, or a plurality of LEDs 322, as the at least one additional light source 310A, 310B, emitting light of at least one wavelength. The at least one LED 322, or the plurality of LEDs 322, further discharges heat generated by its operation. As known to a skilled person, the LED appliance or LED array 320 may co-operate with a reflector array 330 comprising at least one reflector 332, preferably a plurality of reflectors 332. The at least one reflector 332, or the array 330 of a plurality of reflectors 332 correlatable to the LED array 320 has the function of collimating the light emitted by the at least one LED 322, or by the plurality of LEDs 322, to a target, exemplified by a subject or person to be irradiated.

In further preferred embodiments useable in the device 100 of the invention which may be realized with one or two or all other features of the invention and which should not be construed to restrict the invention, the restricted space light appliance cooling system 500, especially the one which is shown in Figure 7D as an exploded view, further comprises a fluorescent covering 340. Said fluorescent covering 340 comprises at least one fluorescent area 342, in the case of a circular shape opening of the corresponding reflectors 332 preferably comprising a plurality of rings 342 as the fluorescent areas 342, said plurality of fluorescent areas or rings 342 even more preferably corresponding to the plurality of reflectors 332 of a reflector array 330, and covering a front edge of at least one reflector 332, preferably the front edges of each reflector 332 of the reflector array 330. Such a plurality of fluorescent areas 342 either consist of a material (e. g. a polymer) provided with (e. g. containing mixed in or containing alloyed in) a dye material excitable by the light emitted by the at least one LED 322 or the plurality of LEDs 322, or is coated with a dye excitable by the light emitted by the at least one LED 322 or the plurality of LEDs 322, so as to emit fluorescent light of the visible wavelength range. The latter feature allows a person viewing to the LED and reflector array to recognize that the LED array 320 with the plurality of LEDs 322 is operating, even if the light itself emitted by the LED / LEDs 322 has a wavelength in the non-visible wavelength range (e. g. is a LED 322 or a plurality of LEDs 322 emitting UV light) and, hence, cannot be discerned by the viewer.

In a number of further preferred embodiments of the restricted space light appliance cooling system 500 useable in the device 100 of the present invention which may be realized with one or two or all other features of the invention and which should not be construed to restrict the invention, the restricted space light appliance cooling system 500, which is especially shown in Figure 7D as an exploded view, may be characterized by further features which, one feature alone or two features together or all features together are those features in detail explained above in relation to the general restricted space cooling system of the first embodiment and which were described above in detail and which, hence, need no second detailed description here. In addition, these additional and, in these cases, preferred embodiments of the said restricted space light appliance cooling system 500 are described above in detail.

In other words: The present invention relating to a radiation-irradiating device 100 for applying directed actinic radiation to a subject / user 200 also comprises a use of a restricted space cooling system 500 described above in detail for providing cooling at a site where heat is, or might be, generated and where only a restricted/confined space is available for accommodating a highly efficient cooling system.

Likewise, the present invention relating to a device 100 for applying directed actinic radiation to a subject / user 200 also comprises a use of a restricted space light appliance cooling system 500 described above in detail for cooling light sources 322 of light appliances 320, preferably for cooling LED appliances 320 including a plurality of simultaneously or sequentially operated LEDs 322; especially for cooling light sources, including one or more LED(s) of a LED array, of a device 100 for irradiating a human body or parts thereof for medical treatment or disease prevention purposes or for cosmetic or wellness purposes.

In a further aspect, the present invention also relates to a method of operating the radiation-irradiating device 100 described above. Specifically, the invention relates to a method of operating the device 100 applying a directed actinic radiation to a subject 200, said method comprising the steps of
- providing a device 100 as claimed in any one of the claims 1 to 9 and as described in detail above; and
- operating at least one of the device's additional actinic light radiation-emitting light sources 310A, 310B by allowing at least one of them to emit directed actinic radiation towards a subject 200 being accommodated on or in said device 100 in a direction forming an acute angle with the longitudinal axis A - A of the device 100.

In further preferred embodiments of the method of the invention which may be realized with one or two or all other features of the invention and which should not be construed to restrict the invention, the operation of the above device 100 is effected to a subject which is a human being.

In other further preferred embodiments of the method useable with the device 100 of the invention which may be realized with one or two or all other features of the invention and which should not be construed to restrict the invention, the operation of at least one, preferably of a plurality of, the device's additional actinic light radiation-emitting light sources 310A, 310B is effected by directing the radiation emitted by said additional light sources in a direction forming an angle with the longitudinal axis A - A of the device 100 in a range of from 8 to 50 degrees (°). Even more preferably, by directing the radiation to the subject / user 200 in an angle of from 15 to 45 degrees, those areas of the subject's /user's body can be provided in high efficiency with the desired actinic radiation, which usually are not targeted by such radiation. The desired cosmetic or wellness effect can thus be achieved, and tanning of the object's / user's scalp and shoulders, for example, can be effected in the same manner as tanning of the user's whole body.

The invention was described above with respect to its general aspects and its preferred aspects as well. However, the invention is not restricted those embodiments described above in detail as preferred and, hence, exemplary embodiments. The scope of the invention is to be determined by the enclosed claims.

### List of Reference Numerals

- 100: Device for an application of directed actinic radiation

- 110: Exposition tunnel
- 111: Head-side longitudinal end of exposition tunnel
- 112: Foot-side longitudinal end of exposition tunnel
- 120: Semi-cylinder barrel-shape first surface
- 130: Inner space
- 140: Second surface
- 141: Semi-cylinder barrel-shape second surface
- 142: Substantially flat (second) surface
- 143: Additional arcuated or substantially flat surface
- 150, 160: Outer spaces

- 170: Light source(s)
- 170u/170l: Upper light sources/lower light sources

- 180, 190: First (upper)/second (lower) parts of the chassis
- 182, 192: Means providing first/second chassis parts 180,190 with power
- 183, 193: Heating/cooling means
- 184, 194: Device operation means, including a processing unit

- 200: subject/user
- 205: User's head
- 207: User's scalp
- 210: User's shoulder(s)
- 215: User's arm(s)
- 220: User's leg(s)
- 300: Further outer space housing additional radiation sources
- 310A, B: Additional radiation sources
- 320: Light source array / LED array
- 321: Light source array (LED array) backing plate of a fixture
- 322: LED / plurality of LEDs
- 330: Reflector array corresponding to the light source / LED array
- 332: Reflector
- 340: Fluorescent covering
- 342: Fluorescent area

- 500: Narrow space (light appliance) cooling system
- 510: Thermal interface
- 520: Heat pipe(s)
- 521: Heat pipe proximal end
- 522: Heat pipe distal end
- 530: Heat exchanger
- 540: Heat dissipation means / fan / cooling jacket
- 550: Heat absorbing medium
- 560: Vent

- A: Logitudinal axis of device 100

## Claims

1. A device (100) for an application of directed actinic radiation to a subject (200), said device (100) comprising:
- an exposition tunnel (110) being capable of surrounding said subject (200) to be exposed to said actinic radiation, said exposition tunnel (110) being formed of one semi-cylinder barrel-shape first surface (120) made of a material substantially permeable to said actinic radiation to be applied to the subject (200), and of at least one second surface (140) made of said material substantially permeable to said actinic radiation to be applied to the subject (200), said at least one second surface (140) being capable of complementing the first surface (120) in forming said exposition tunnel (110);
- the surfaces (120, 140) of said exposition tunnel (110) separating an inner space (130) where said subject (200) is exposed to said actinic radiation, from outer spaces (150, 160) where a plurality of radiation sources (170) capable of emitting actinic radiation through said surfaces (120, 140) are mounted;
- said outer spaces (150, 160) being housed in at least first and second parts of a chassis (180, 190) of the device (100) and comprise
means for fixedly holding, and electrically and electronically operating, said plurality of radiation sources (170);
means (182, 192) providing said at least first and second parts of the chassis (180, 190) with electric power;
means (183, 193) capable of ventilating, heating and/or cooling the device (100); and
means (184, 194) for the operation of the device (100), including a processing unit;
- said first and second parts of the chassis (180, 190) of the device (100) being configured to complement each other in forming the outer shape of the device (100) by being configured to hingedly move away from, or towards, each other;
said device (100) further comprising at least one further outer space (300) separated from said inner space (130) and located at either one or both of the longitudinal ends (111, 112) of the exposition tunnel (110), said at least one further outer space (300) housing a plurality of additional radiation sources (310A, 310B) configured to emit radiation towards the subject (200) in a direction forming an acute angle with a longitudinal axis (A - A) of the device (100), and
at least one cooling means for the plurality of additional radiation sources (310A, 310B),
**characterized in**
**that** the additional radiation sources (310A, 310B) comprise LEDs (322), and
**that** said at least one cooling means for the plurality of additional radiation sources (310A, 310B) comprises at least one narrow space light appliance cooling system (500) comprising heat pipes (520) for cooling the plurality of additional radiation sources (310A, 310B).

2. The device (100) as claimed in claim 1, the device (100) being configured to irradiate a human being to be exposed to said actinic radiation.

3. The device (100) as claimed in claim 1 or claim 2, wherein said exposition tunnel (110) has a longitudinal axis (A - A) arranged horizontally, or wherein said exposition tunnel (110) has a longitudinal axis (A - A) arranged vertically.

4. The device (100) as claimed in any one or more of the claims 1 to 3, wherein said exposition tunnel (110) is formed of said one semi-cylinder barrel-shape surface (120) and of a at least one substantially flat surface (142), or wherein said exposition tunnel (110) is formed of said one semi-cylinder barrel-shape surface (120) and of a second semi-cylinder barrel-shape surface (141).

5. The device as claimed in any one or more of the claims 1 to 4, wherein said at least one further outer space (300) is one further outer space (300) disposed at the head-side longitudinal end (111) of the exposition tunnel (110).

6. The device as claimed in claim 5, wherein said one further outer space (300) housing the plurality of additional radiation sources (310A, 310B) capable of emitting radiation towards the subject's scalp (207) and shoulders (210).

7. The device (100) as claimed in any one or more of the claims 1 to 6, wherein the plurality of additional radiation sources (310A, 310B) comprising LEDs (322) is further selected from the group consisting of high-pressure lamps, medium pressure lamps, low-pressure lamps, LEDs and combinations thereof, preferably wherein the plurality of additional radiation sources (310A, 310B) comprising LEDs (322) is further selected from the group consisting of high-pressure lamps, LEDs and combinations thereof.

8. The device (100) as claimed in any one or more of the claims 1 to 7, further comprising at least one reflector (322) or a plurality of reflectors (322) forming a reflector array (330) for the plurality of additional radiation sources (310A, 310B) to collimate the radiation emitted by said plurality of additional radiation sources (310A, 310B).

9. The device (100) as claimed in claim 3, said device (100) further comprising a fluorescent covering (340) comprising at least one fluorescent area (342), preferably comprising a plurality of fluorescent rings (342), said at least one fluorescent ring (342) or plurality of fluorescent rings (342) even more preferably corresponding to the plurality of reflectors (332) of the reflector array (330), and covering a front edge of at least one reflector (332), preferably the front edge of each reflector (332) of the reflector array (330), and provided with, mixed with, alloyed with or coated with a dye, the dye being excitable by the light emitted by the at least one LED (322) or the plurality of LEDs (322) to emit fluorescent light of the visible wavelength range.

10. The device (100) as claimed in any one or more of the claims 1 to 9, wherein the acute angle of the directed radiation with the device's longitudinal axis (A - A) is within a range of from 8 to 50 degrees, preferably within a range of from 15 to 45 degrees.

11. A non-therapeutical method of operating a device (100) applying a directed actinic radiation to a subject (200), said method comprising the steps of
- providing a device (100) as claimed in any one of the claims 1 to 9; and
- operating at least one of the device's additional actinic light radiation-emitting light sources (310A, 310B) by allowing at least one of them to emit directed actinic radiation towards a subject (200) being accommodated on or in said device (100) in a direction forming an acute angle with the longitudinal axis (A - A) of the device (100).

12. The non-therapeutical method as claimed in claim 11, wherein a human being (200) is exposed to said actinic radiation.

13. The non-therapeutical method as claimed in claim 11 or claim 12, wherein the acute angle of the directed radiation with the device's longitudinal axis (A - A) is within a range of from 8 to 50 degrees, preferably within a range of from 15 to 45 degrees.

## Patentansprüche

1. Vorrichtung (100) für eine Anwendung von gerichteter aktinischer Strahlung an einem Subjekt (200), wobei die Vorrichtung (100) Folgendes umfasst:
- einen Expositionstunnel (110), der in der Lage ist, das Subjekt (200), das der aktinischen Strahlung ausgesetzt werden soll, zu umgeben, wobei der Expositionstunnel (110) aus einer halbzylindrischen, fassförmigen ersten Oberfläche (120), die aus einem für die aktinische Strahlung, die auf das Subjekt (200) angewandt werden soll, im Wesentlichen durchlässigen Material hergestellt ist, und aus wenigstens einer zweiten Oberfläche (140) ausgebildet ist, die aus dem für die aktinische Strahlung, die auf das Subjekt (200) angewandt werden soll, im Wesentlichen durchlässigen Material hergestellt ist, wobei die wenigstens zweite Oberfläche (140) zum Ergänzen der ersten Oberfläche (120) bei dem Ausbilden des Expositionstunnels (110) in der Lage ist;
- wobei die Oberflächen (120, 140) des Expositionstunnels (110), die einen inneren Raum (130), in dem das Subjekt (200) der aktinischen Strahlung ausgesetzt ist, von äußeren Räumen (150, 160) trennen, in denen mehrere Strahlungsquellen (170) angebracht sind, die in der Lage sind, aktinische Strahlung durch die Oberflächen (120, 140) zu emittieren;
- wobei die äußeren Räume (150, 160), die in wenigstens einem ersten und einem zweiten Teil einer Grundplatte (180, 190) der Vorrichtung (100) untergebracht sind; und
Mittel zum festen Halten und elektrischen und elektronischen Betreiben der mehreren Strahlungsquellen (170); Mittel (182, 192), die den wenigstens ersten und zweiten Teil der Grundplatte (180, 190) mit elektrischer Energie versorgen;
Mittel (183, 193), die in der Lage sind, die Vorrichtung (100) zu lüften, zu erwärmen und/oder zu kühlen; und
Mittel (184, 194) für den Betrieb der Vorrichtung (100), die eine Verarbeitungseinheit enthält, umfassen;
- wobei der ersten und der zweite Teil der Grundplatte (180, 190) der Vorrichtung (100) konfiguriert sind, um sich bei dem Ausbilden der äußeren Form der Vorrichtung (100) zu ergänzen, indem sie konfiguriert sind, um sich schwenkbar voneinander weg oder zueinander hin zu bewegen;
wobei die Vorrichtung (100) ferner wenigstens einen weiteren äußeren Raum (300), der von dem inneren Raum (130) getrennt ist und sich an einem oder beiden der Längsenden (111, 112) des Expositionstunnels (110) befindet, umfasst,
wobei der wenigstens eine weitere äußere Raum (300) mehrere zusätzliche Strahlungsquellen (310A, 310B) aufnimmt, die konfiguriert sind, um die Strahlung zu dem Subjekt (200) in einer Richtung zu emittieren, die einen spitzen Winkel mit einer Längsachse (A-A) der Vorrichtung (100) ausbildet, und
wenigstens ein Kühlungsmittel für die mehreren zusätzlichen Strahlungsquellen (310A, 310B),
**dadurch gekennzeichnet,**
**dass** die zusätzlichen Strahlungsquellen (310A, 310B) LEDs (322) enthalten, und
**dass** das wenigstens eine Kühlungsmittel für die mehreren zusätzlichen Strahlungsquellen (310A, 310B) wenigstens ein Lichtgerätkühlungssystem (500) auf engem Raum umfasst, das Heatpipes (520) zum Kühlen der mehreren zusätzlichen Strahlungsquellen (310A, 310B) umfasst.

2. Vorrichtung (100) nach Anspruch 1, wobei die Vorrichtung (100) konfiguriert ist, um einen Menschen zu bestrahlen, der der aktinischen Strahlung ausgesetzt werden soll.

3. Vorrichtung (100) nach Anspruch 1 oder 2, wobei der Expositionstunnel (110) eine horizontal angeordnete Längsachse (A-A) aufweist oder wobei der Expositionstunnel (110) eine vertikal angeordnete Längsachse (A-A) aufweist.

4. Vorrichtung (100) nach einem oder mehreren der Ansprüche 1 bis 3, wobei der Expositionstunnel (110) aus der einen halbzylindrischen fassförmigen Oberfläche (120) und aus einer wenigstens einen im Wesentlichen flachen Oberfläche (142) ausgebildet ist, oder wobei der Expositionstunnel (110) aus der einen halbzylindrischen fassförmigen Oberfläche (120) und einer zweiten halbzylindrischen fassförmigen Oberfläche (141) ausgebildet ist.

5. Vorrichtung nach einem oder mehreren der Ansprüche 1 bis 4, wobei der wenigstens eine weitere äußere Raum (300) ein weiterer äußerer Raum (300) ist, der an dem kopfseitigen Längsende (111) des Expositionstunnels (110) angeordnet ist.

6. Vorrichtung nach Anspruch 5, wobei der eine weitere äußere Raum (300) die mehreren zusätzlichen Strahlungsquellen (310A, 310B) aufnimmt, die in der Lage sind, Strahlung zu der Kopfhaut (207) und den Schultern (210) des Subjekts zu emittieren.

7. Vorrichtung (100) nach einem oder mehreren der Ansprüche 1 bis 6, wobei die mehreren zusätzlichen Strahlungsquellen (310A, 310B), die LEDs (322) umfassen, ferner aus der Gruppe ausgewählt sind, bestehend aus Hochdrucklampen, Mitteldrucklampen, Niederdrucklampen, LEDs und Kombinationen davon, vorzugsweise wobei die mehreren zusätzlichen Strahlungsquellen (310A, 310B), die LEDs (322) umfassen, ferner aus der Gruppe ausgewählt sind, bestehend aus Hochdrucklampen, LEDs und Kombinationen davon.

8. Vorrichtung (100) nach einem oder mehreren der Ansprüche 1 bis 7, ferner umfassend wenigstens einen Reflektor (322) oder mehrere Reflektoren (322), die eine Reflektoranordnung (330) für die mehreren zusätzlichen Strahlungsquellen (310A, 310B) ausbilden, um die von den mehreren zusätzlichen Strahlungsquellen (310A, 310B) emittierte Strahlung zu kollimieren.

9. Vorrichtung (100) nach Anspruch 8, wobei die Vorrichtung (100) ferner einen fluoreszierende Belag (340) umfasst, der wenigstens einen fluoreszierenden Bereich (342) umfasst, und vorzugsweise mehrere fluoreszierende Ringe (342) umfasst, wobei der wenigstens eine fluoreszierende Ring (342) oder die mehreren fluoreszierenden Ringe (342) noch bevorzugter den mehreren Reflektoren (332) der Reflektoranordnung (330) entsprechen, und eine Vorderkante wenigstens eines Reflektors (332), vorzugsweise die Vorderkante jedes Reflektors (332) der Reflektoranordnung (330), abdecken und mit einem Farbstoff versehen, gemischt, legiert oder beschichtet sind, wobei der Farbstoff durch das von der wenigstens einen LED (322) oder den mehreren LEDs (322) emittierte Licht angeregt werden kann, um fluoreszierendes Licht des sichtbaren Wellenlängenbereichs zu emittieren.

10. Vorrichtung (100) nach einem oder mehreren der Ansprüche 1 bis 9, wobei der spitze Winkel der gerichteten Strahlung mit der Längsachse (A-A) der Vorrichtung in einem Bereich von 8 bis 50 Grad liegt, vorzugsweise in einem Bereich von 15 bis 45 Grad.

11. Nichttherapeutisches Verfahren zum Betreiben einer Vorrichtung (100), die eine gerichtete aktinische Strahlung auf ein Subjekt (200) anwendet, wobei das Verfahren die folgenden Schritte umfasst:
- Bereitstellen einer Vorrichtung (100) nach einem der Ansprüche 1 bis 9; und
- Betreiben wenigstens einer der zusätzlichen eine aktinische Lichtstrahlung emittierenden Lichtquellen (310A, 310B) der Vorrichtung, indem wenigstens einer von ihnen ermöglicht wird, gerichtete aktinische Strahlung zu einem Subjekt (200), das auf oder in der Vorrichtung (100) aufgenommen wird, in einer Richtung zu emittieren, die einen spitzen Winkel mit der Längsachse (A-A) der Vorrichtung (100) ausbildet.

12. Nichttherapeutisches Verfahren nach Anspruch 11, wobei ein Mensch (200) der aktinischen Strahlung ausgesetzt wird.

13. Nichttherapeutisches Verfahren nach Anspruch 11 oder 12, wobei der spitze Winkel der gerichteten Strahlung mit der Längsachse (A-A) der Vorrichtung in einem Bereich von 8 bis 50 Grad liegt, vorzugsweise in einem Bereich von 15 bis 45 Grad.

## Revendications

1. Dispositif (100) d'application de radiation actinique dirigée à un sujet (200), ledit dispositif (100) comprenant :
- un tunnel d'exposition (110) apte à entourer ledit sujet (200) à exposer à ladite radiation actinique, ledit tunnel d'exposition (110) étant formé d'une première surface (120) en forme de tonneau semi-cylindrique constituée d'un matériau sensiblement perméable à ladite radiation actinique à appliquer au sujet (200), et d'au moins une seconde surface (140) constituée dudit matériau sensiblement perméable à ladite radiation actinique à appliquer au sujet (200), ladite au moins une seconde surface (140) étant apte à compléter la première surface (120) dans la formation dudit tunnel d'exposition (110) ;
- les surfaces (120, 140) dudit tunnel d'exposition (110) séparant un espace intérieur (130) où ledit sujet (200) est exposé à ladite radiation actinique d'espaces extérieurs (150, 160) où une pluralité de sources (170) de radiation aptes à émettre une radiation actinique à travers lesdites surfaces (120, 140) sont montées ;
- lesdits espaces extérieurs (150, 160) étant logés dans des au moins première et seconde parties d'un châssis (180, 190) du dispositif (100) et comprenant
des moyens de maintenir à demeure, et d'actionner électriquement et électroniquement, ladite pluralité de sources (170) de radiation ;
des moyens (182, 192) alimentant lesdites au moins première et seconde parties du châssis (180, 190) en énergie électrique ; des moyens (183, 193) aptes à ventiler, chauffer et/ou refroidir le dispositif (100) ; et
des moyens (184, 194) d'actionnement du dispositif (100), dont une unité de traitement ;
- lesdites première et seconde parties du châssis (180, 190) du dispositif (100) étant conçues pour se compléter l'une l'autre dans la formation de la forme extérieure du dispositif (100) en étant conçues pour s'éloigner ou se rapprocher de manière articulée l'une de l'autre ;
ledit dispositif (100) comprenant en outre au moins un autre espace extérieur (300) séparé dudit espace intérieur (130) et situé à l'une ou aux deux extrémités longitudinales (111, 112) du tunnel d'exposition (110), ledit au moins un autre espace extérieur (300) logeant une pluralité de sources (310A, 310B) de radiation supplémentaires conçues pour émettre une radiation vers le sujet (200) dans une direction formant un angle aigu avec un axe longitudinal (A - A) du dispositif (100), et
au moins un moyen de refroidissement pour la pluralité de sources (310A, 310B) de radiation supplémentaires,
**caractérisé en ce**
**que** les sources (310A, 310B) de radiation supplémentaires comprennent des DEL (322), et
**que** ledit au moins un moyen de refroidissement pour la pluralité de sources (310A, 310B) de radiation supplémentaires comprend au moins un système (500) de refroidissement d'appareil d'éclairage d'espace étroit comprenant des caloducs (520) servant à refroidir la pluralité de sources (310A, 310B) de radiation supplémentaires.

2. Dispositif (100) selon la revendication 1, le dispositif (100) étant configuré pour irradier un être humain à exposer à ladite radiation actinique.

3. Dispositif (100) selon la revendication 1 ou la revendication 2, dans lequel ledit tunnel d'exposition (110) présente un axe longitudinal (A - A) agencé horizontalement, ou dans lequel ledit tunnel d'exposition (110) présente un axe longitudinal (A - A) agencé verticalement.

4. Dispositif (100) selon l'une quelconque, ou plusieurs, des revendications 1 à 3, dans lequel ledit tunnel d'exposition (110) est formé de ladite surface (120) en forme de tonneau semi-cylindrique et d'au moins une surface sensiblement plate (142), ou dans lequel ledit tunnel d'exposition (110) est formé de ladite surface (120) en forme de tonneau semi-cylindrique et d'une seconde surface (141) en forme de tonneau semi-cylindrique.

5. Dispositif selon l'une quelconque, ou plusieurs, des revendications 1 à 4, dans lequel ledit au moins un autre espace extérieur (300) est un autre espace extérieur (300) disposé à l'extrémité longitudinale (111) côté tête du tunnel d'exposition (110).

6. Dispositif selon la revendication 5, dans lequel ledit autre espace extérieur (300) loge la pluralité de sources (310A, 310B) de radiation supplémentaires aptes à émettre une radiation vers le cuir chevelu (207) et les épaules (210) du sujet.

7. Dispositif (100) selon l'une quelconque, ou plusieurs, des revendications 1 à 6, la pluralité de sources (310A, 310B) de radiation supplémentaires comprenant des DEL (322) étant en outre sélectionnée dans le groupe constitué de lampes haute pression, de lampes moyenne pression, de lampes basse pression, de DEL et de leurs combinaisons, la pluralité de sources (310A, 310B) de radiation supplémentaires comprenant des DEL (322) étant de préférence en outre choisie dans le groupe constitué de lampes haute pression, de DEL et de leurs combinaisons.

8. Dispositif (100) selon l'une quelconque, ou plusieurs, des revendications 1 à 7, comprenant en outre au moins un réflecteur (322) ou une pluralité de réflecteurs (322) formant un réseau (330) de réflecteurs pour la pluralité de sources (310A, 310B) de radiation supplémentaires pour collimater la radiation émise par ladite pluralité de sources (310A, 310B) de radiation supplémentaires.

9. Dispositif (100) selon la revendication 8, ledit dispositif (100) comprenant en outre une couverture fluorescente (340) comprenant au moins une zone fluorescente (342), comprenant de préférence une pluralité d'anneaux fluorescents (342), ledit au moins un anneau fluorescent (342) ou ladite pluralité d'anneaux fluorescents (342) correspondant encore plus préférentiellement à la pluralité de réflecteurs (332) du réseau (330) de réflecteurs, et recouvrant un bord avant d'au moins un réflecteur (332), de préférence le bord avant de chaque réflecteur (332) du réseau (330) de réflecteurs, et pourvu de, mélangé à, allié à, ou revêtu d'un colorant, le colorant étant excitable par la lumière émise par l'au moins une DEL (322) ou la pluralité de DEL (322) de manière à émettre une lumière fluorescente de la gamme de longueurs d'onde visible.

10. Dispositif (100) selon l'une quelconque, ou plusieurs, des revendications 1 à 9, dans lequel l'angle aigu de la radiation dirigée et de l'axe longitudinal (A - A) du dispositif est compris dans une plage de 8 à 50 degrés, de préférence compris dans une plage de 15 à 45 degrés.

11. Procédé non thérapeutique d'actionnement d'un dispositif (100) appliquant une radiation actinique dirigée à un sujet (200), ledit procédé comprenant les étapes consistant à
- fournir un dispositif (100) selon l'une quelconque des revendications 1 à 9 ; et à
- actionner au moins l'une des sources (310A, 310B) de lumière électroluminescente actinique supplémentaires du dispositif en permettant à au moins l'une d'elles d'émettre une radiation actinique dirigée vers un sujet (200) accueilli sur ou dans ledit dispositif (100) dans une direction formant un angle aigu avec l'axe longitudinal (A - A) du dispositif (100).

12. Procédé non thérapeutique selon la revendication 11, dans lequel un être humain (200) est exposé à ladite radiation actinique.

13. Procédé non thérapeutique selon la revendication 11 ou la revendication 12, dans lequel l'angle aigu de la radiation dirigée et de l'axe longitudinal (A - A) du dispositif est compris dans une plage de 8 à 50 degrés, de préférence compris dans une plage de 15 à 45 degrés.
